Europäisches Patentamt

(19) European Patent Office

Office européen des brevets

(11) Numéro de publication: **0 034 334**
**B1**

(12) FASCICULE DE BREVET EUROPEEN

(45) Date de publication du fascicule du brevet:
09.02.83

(51) Int. Cl.³: **C 07 C 69/07**, C 07 C 69/24,
C 07 C 69/88, C 07 C 69/533,
A 23 L 1/22, A 61 K 7/46

(21) Numéro de dépôt: 81100969.5

(22) Date de dépôt: 11.02.81

(54) Esters de 1,3-diméthyl-but-3-ène-1-yle, leur utilisation en tant qu'ingrédients parfumants ou aromatisants, composition parfumante ou aromatisante contenant au moins l'un desdits esters.

(30) Priorité: 13.02.80 CH 1162/80

(43) Date de publication de la demande:
26.08.81 Bulletin 81/34

(45) Mention de la délivrance du brevet:
09.02.83 Bulletin 83/6

(84) Etats contractants désignés:
CH DE FR GB LI NL

(56) Documents cités:
WO-A-79/00588
GB-A-566 497
BULLETIN DE LA SOCIETE CHIMIQUE DE FRANCE, 1956 Paris FR J. COLONGE et al.: »Oxydation par l'anhydride sélénieux. II. Alcools de constitutions diverses«, pages 188—194
JOURNAL OF ORGANIC CHEMISTRY, Vol. 38, no. 12, 15-06-1973, Washington, US A.I. MEYERS et al.: »The synthesis of alpha-branched ketones from dihydro-1,3-oxazines via the ketenimine intermediate. alpha-Substituted ketones from a stable ketenimine«, pages 2129—2136

(73) Titulaire: FIRMENICH SA, Case postale 239,
CH-1211 Genève 8 (CH)

(72) Inventeur: Sundt, Erling, 12, ch. de la Tour de Pinchat,
CH-1234 Vessy/Ge (CH)
Inventeur: Schenk, Walter, 3, avenue Weber,
CH-1208 Geneve (CH)
Inventeur: Chappaz, Roger, 10, quai des Arénières,
CH-1205 Geneve (CH)
Inventeur: Joyeux, Michel, 23, ch. des Recluses,
CH-1213 Grand-Lancy/Ge (CH)

(74) Mandataire: Schmied-Kowarzik, Volker, Dr. et al,
Siegfriedstrasse 8, D-8000 München 40 (DE)

CHEMICAL ABSTRACTS, vol. 35, no. 6, 20-03-1941, colonne 17 654 Columbus, Ohio, US J. KENYON et al.: »Composition of the Pondorff-Meerwein reduction product of mesityl oxide«

0 034 334

Esters de 1,3-diméthyl-but-3-ène-1-yle, leur utilisation en tant qu'ingrédients parfumants
ou aromatisants, composition parfumante ou aromatisante contenant au moins l'un desdits esters

La présente invention a trait au domaine de la parfumerie et des arômes, en pariculier elle concerne certains esters de 1,3-diméthyl-but-3-ène-1-yle ainsi qu'un procédé pour améliorer, modifier ou renforcer les propriétés odorantes de parfums et produits parfumés ainsi que les propriétés gustatives d'aliments ou boissons, lequel procédé est caractérisé en ce qu'on ajoute auxdits parfums, produits parfumés, aliments ou boissons l'un des esters mentionnés. L'invention a également trait à une composition parfumante ou aromatisante contenant en tant qu'ingrédient actif au moins un desdits esters.

Parmi la variété de corps synthétiques mis à la disposition du parfumeur ou de l'aromatiseur figurent plusieurs esters dérivés d'acides aliphatiques carboxyliques et d'alcools aliphatiques inférieurs.

Dans le but d'élargir encore davantage l'éventail des ingrédients offerts dans cette classe de composés de façon à pouvoir permettre la création de compositions nouvelles aux nuance odorantes inédites, nous avons réalisé la synthèse d'une série de composés nouveaux. Il s'agit en l'occurence d'esters dérivés du 1,3-diméthyl-but-3-ène-1-ol, en particulier des composés que voici:

- formiate de 1,3-diméthyl-but-3-ène-1-yle,
- propionate de 1,3-diméthyl-but-3-ène-1-yle,
- butyrate de 1,3-diméthyl-but-3-ène-1-yle,
- isobutyrate de 1,3-diméthyl-but-3-ène-1-yle,
- isovalérianate de 1,3-diméthyl-but-3-ène-1-yle,
- tiglate de 1,3-diméthyl-but-3-ène-1-yle, et
- salicylate de 1,3-diméthyl-but-3-ène-1-yle.

De tels composés présentent un intérèt tout particulier pour l'industrie de la parfumerie et celle des arômes; ils développent en effet des notes variées de type vert, fleuri, ou parfois fruité. L'odeur développée par certains d'entre eux rappelle celle possédée par la camomille, notamment dans ses notes de tête.

De par la variété de leurs propriétés olfactives, les esters de l'invention peuvent être utilisés dans bon nombre de compositions ou bases parfumantes de nature variée, leurs caractères se mariant aisément avec les ingrédients parfumants courants; leur domaine d'utilisation est donc assez étendu.

Dans la parfumerie, les proportions dans lesquelles lesdits composés de l'invention peuvent produire des effets intéressants varient dans une large gamme de valeurs. C'est ainsi que des proportions de l'ordre de 5 à 20% en poids, par rapport au poids de la composition dans laquelle ils sont incorporés, ont été jugées comme satisfaisantes dans la plupart des applications considérées.

Bien entendu, de telles concentrations peuvent varier et avoir des valeurs se situant en deçà ou au delà des limites indiquées. Tel est le cas, par exemple, dans les applications destinées au parfumage des produits d'entretien, des détergents, des cosmétiques ou des savons, où des concentrations de l'ordre de 0,1 à 0,5% en poids peuvent déjà produire des effets odorants intéressants.

Dans le domaine des arômes, les composés de l'invention développent des notes gustatives herbales, fleuries, rosées, résineuses, fruitées pouvant rappeler la camomille.

Un intérêt particulier est présenté par les esters propionique, isobutyrique et tiglique.

Dans le domaine des arômes, les composés de l'invention peuvent être utilisés à des concentrations variables. Des proportions de l'ordre de 1 à 50 ppm, plus particulièrement de 10 ppm (parties par million) par rapport au poids total du produit aromatisé peuvent convenablement être utilisées. Parmi les esters définis plus haut, le salicylate de 1,3-diméthyl-but-3-ène-1-yle est résulté d'une puissance très particulière; il peut dont être utilisé à des concentrations inférieures à celles indiquées ci-dessus, par exemple de l'ordre de 0,1 à 0,5 ppm.

Les esters de l'invention peuvent être utilisés isolément ou, plus fréquemment, en combinaison avec d'autres ingrédients parfumants, respectivement aromatisants d'origine naturelle ou synthétique, avec des solvants ou des supports.

Un intérêt particulier est représenté par l'isobutyrate, le tiglate, et le salicylate.

Lesdits esters peuvent être préparés à partir du 1,3-diméthyl-but-3-ène-1-ol conformément à des procédés usuels. Les méthodes suivies sont indiquées ci-après par le détail.

## Préparation des esters propionique, butyrique et isobutyrique

0,2 Moles du chlorure d'acide approprié (chlorure de propionyle, butyryle ou isobutyryle) ont été ajoutées goutte à goutte à un mélange maintenu à 0/+5° de 1,3-diméthyl-but-3-ène-1-ol (0,2 mole) et diéthylaniline (0,22 mole).

Après l'introduction, le mélange réactionnel a été maintenu sous agitation à température ambiante pendant 2 h tout en y ajoutant 50 ml d'éther isopropylique, puis 0,04 moles du chlorure d'acide approprié afin de compléter la réaction.

**0 034 334**

Le mélange est ensuite chauffé pendant 2 h à 40–50° et laissé sous agitation pendant la nuit à température ambiante, puis refroidi à environ 5°. 50 ml d'acide sulfurique à 5% ont ensuite été ajoutés au mélange et la phase organique a été lavée 1 fois avec de l'eau, 2 fois avec une solution de carbonate de sodium à 10% et enfin 2 fois avec une solution aqueuse concentrée de NaCl. La phase organique a été séchée sur du $Na_2SO_4$, concentrée et distillée pour fournir l'ester désiré.

ester propionique:
 rendement 70%; Eb. 59°/1,73 · $10^3$ Pascal
SM: m/e = 57, 82, 29, 67, 41, 101, 112, 141, 119.

ester butyrique:
 rendement 70% environ; Eb. 75°/1,73 · $10^3$ Pascal
SM: m/e = 71, 43, 82, 55, 27, 115, 89, 126, 141, 155, 105.

ester isobutyrique:
 rendement 70% environ; Eb. 60°/1,33 · $10^3$ Pascal
SM: m/e = 71, 43, 82, 55, 27, 115, 126, 155.

### Préparation de l'ester formique

46 g (1 mole) d'acide formique ont été ajoutés goutte à goutte à 50 g (0,5 mole) de 1,3-diméthyl-but-3-ène-1-ol sous vigoureuse agitation et le mélange a été laissé au repos pendant 2 h à température ambiante, puis il a été maintenu 4 h à 60°. Le mélange a été ensuite dilué au pentane et la phase organique a été lavée deux fois à l'eau, au bicarbonate de sodium et enfin à l'eau jusqu'à neutralité. Après filtration sur une colonne de $SiO_2$ et évaporation, on a distillé sous pression réduite à l'aide d'un appareil de type Widmer pour fournir 17,7 g du produit désiré:

Eb. 110°/9,71 · $10^4$ Pascal

RMN: 1,22 et 1,33 (3H, 2s); 1,78 (3H, s); 2,3 (2H, q); 4,78–5,25 (3H, m); 8,0 (1H, s) δ ppm.

### Préparation de l'ester isovalérianique

20,9 g de chlorure d'isovalérianyle (0,2 mole) ont été ajoutés sous agitation en 45 minutes à une solution de 20 g (0,2 mole) de 1,3-diméthyl-but-3-ène-1-ol dans 60 ml d'éther absolu et 23,7 g (0,3 mole) de pyridine absolue. Le mélange est ensuite maintenu au reflux pendant 1 h, puis refroidi à température ambiante. Après addition de 100 ml d'eau glacée, extraction à l'éther sulfurique (2x) et traitement habituel d'acidification, lavage et neutralisation des extraits organiques, on a obtenu par évaporation et distillation l'ester désiré ayant Eb. 71°/1,33 · $10^3$ Pascal (rendement 66,8%).

SM: m/e = 129, 103, 85, 82, 67, 57, 41, 29
RMN: 0,83 et 0,95 (6H, 2s); 1,10 et 1,21 (3H, 2s); 1,70 (3H, 1s); 2,12 (5H, m); 4,70 (2H, m); 5,05 (1H, q) δ ppm.

### Préparation de l'ester tiglique (3-méthyl-trans-but-2-énoïque)

13,5 g (0,113 mole) de chlorure de 3-méthyl-trans-but-2-énoyle ont été ajoutés en 5 minutes à une solution de 10 g (0,1 mole) de 1,3-diméthyl-but-3-ène-1-ol dans 115 ml de toluène anhydre. Le mélange de réaction a été ensuite chauffé au reflux pendant $3^1/_2$ h et, après refroidissement, il a été mélangé avec 45 ml d'une solution aqueuse saturée de $NaHCO_3$, puis agité à température ambiante pendant 30 minutes.

La phase organique séparée a été soumise aux traitements habituels de lavage et neutralisation. Après évaporation des parties volatiles, on soumet le résidu obtenu à deux distillations fractionnées successives à l'aide d'une colonne Vigreux et d'une colonne de type Fischer, respectivement, pour fournir le produit désiré ayant Eb. 85°/1,33 · $10^3$ Pascal; rendement 44%.

SM: m/e = 127, 101, 83, 82, 67, 55, 39, 29
RMN: 1,18 et 1,28 (3H, d); 1,72–1,80 (9H); 2,20–2,36 (2H); 4,70 (2H, s); 4,92–5,25 (1H, q); 6,80 (1H, m) δ ppm.

3

0 034 334

## Préparation de l'ester salicylique

Cet ester a été préparé comme indiqué plus haut pour l'ester isovalérianique en utilisant 1,0 kg (10 moles) de 1,3-diméthyl-but-3-ène-1-ol et du chlorure de salicycle obtenu par chloruration de 1,380 kg (10 moles) d'acide salicylique par 1,190 kg (10 moles) de chlorure de thionyle en présence de pyridine.

Le salicylate de 1,3-diméthyl-but-3-ène-1-yle obtenu montrait Eb. 110°/1,33 Pascal; 1,520 kg; rendement 69%.

RMN: 1,3 et 1,4 (4H, 2s); 1,60 (1H, 1d); 1,76 (3H, 1s); 2,37 (2H, q); 3,78 (2H, s); 5,35 (1H, q); 6,8 – 7,87 (4H, m) δ ppm.

Nous avons observé que les esters saturés correspondants, à savoir les ésters de 1,3-diméthyl-butyle présentent également des propriétés organoleptiques intéressantes, quoique moins prononcées. Lesdits esters sont préparés à partir de 1,3-diméthyl-butanol selon les méthodes d'estérification usuelles.

Le 1,3-diméthyl-but-3-ène-1-ol, utilisé comme produit de départ dans les méthodes de préparation décrites ci-dessus, peut être obtenu conformément à des méthodes connues, par exemple suivant la demande japonaise no. 76 70708 [voir Chem. Abstr. 85, 123 338 d (1976)].

Les températures indiquées plus haut sont données en degrés centigrades et les abréviations ont le sens usuel dans l'art.

### Exemple 1

Les composés dont il est question dans la présente invention ont été soumis à une évaluation olfactive de la part d'un groupe d'experts. Le tableau suivant résume les résultats d'une telle évaluation.

| Ester de 1,3-diméthyl-but-3-ène-l-yle | Evaluation olfactive |
| --- | --- |
| formiate | fugace, vert |
| propionate | vert, fleuri-fruité |
| butyrate | fleuri-fruité |
| isobutyrate | excellente note fleurie et fruitée, rappelle la note de tête de la camomille |
| isovalérianate | fleuri-fruité |
| tiglate | bonne note fleurie-fruitée, rappelle certains aspects de la camomille |
| salicylate | fleuri, vert |

### Exemple 2

On a préparé une composition parfumante de base de type fleuri et fruité en mélangeant les ingrédients suivants (parties en poids):

| | |
| --- | --- |
| alcool phényléthylique | 100 |
| salicylate de cis-hex-3-énol | 80 |
| acétate de triméthylhexyle | 80 |
| acétate de benzyle | 80 |
| linalol | 60 |
| héliotropine | 50 |
| hydroxycitronellol | 50 |
| acétate de citronellyle | 40 |
| citronellol gauche | 40 |
| aldéhyde undécylénique 10%*) | 40 |
| 1,1-diméthyl-4-acétyl-6-tert-butyl-indane | 30 |

4

| | |
|---|---|
| aldéhyde α-amylcinnamique | 30 |
| α-isométhyl-ionone | 30 |
| α-damascone à 10%*) | 20 |
| acétate de styrallyle | 20 |
| 4-isopropyl-cyclohexyl-méthanol | 10 |
| salicylate d'amyle | 20 |
| caproate d'allyle à 10%*) | 20 |
| Hédione¹)® | 10 |
| aldéhyde anisique | 5 |
| β-damascenone à 1%*) | 5 |
| α-méthyl-p-tert-butyl-hydro-cinnamaldéhyde | 20 |
| | 840 |

\*) dans diéthyl phthalate
¹) Origine: Firmenich SA, Genève, Suisse

En ajoutant à la nouvelle composition ainsi obtenue 10 g d'isobutyrate de 1,3-diméthyl-but-3-ène-1-yle, on obtient une composition parfumante possédant une note de type camomille. Une telle composition est parfaitement adaptée au parfumage de produits destinés aux soins capillaires, des shampoings par exemple.

Exemple 3

Les composés dont il est question dans la présente invention ont été soumis à une évaluation gustative de la part d'un groupe d'experts. Le tableau suivant résume les résultats d'une telle évaluation:

| Ester de 1,3-diméthyl-but-3-ène-l-yle | Evaluation gustative |
|---|---|
| propionate | herbal, camomille, légèrement fleuri-rosé |
| butyrate | gras |
| isobutyrate | herbal, résineux |
| isovalérianate | fruité |
| tiglate | herbal, résineux |
| salicylate | fleuri |

Les composés examinés ont été évalués dans de l'eau de source à une concentration moyenne de 10 ppm (parties par million) à l'exception du salicylate qui a été évalué à 0,3 ppm.

**Abrege**

Certains esters de 1,3-diméthyl-but-3-ène-1-yle, à savoir le

— formiate de 1,3-diméthyl-but-3-ène-1-yle,
— propionate de 1,3-diméthyl-but-3-ène-1-yle,
— butyrate de 1,3-diméthyl-but-3-ène-1-yle,
— isobutyrate de 1,3-diméthyl-but-3-ène-1-yle,
— isovalérianate de 1,3-diméthyl-but-3-ène-1-yle,
— tiglate de 1,3-diméthyl-but-3-ène-1-yle, et le
— salicylate de 1,3-diméthyl-but-3-ène-1-yle

possèdent des propriétés organoleptiques intéressantes et peuvent de ce fait être utilisés dans l'industrie des arômes et, plus particulièrement celle de la parfumerie. Ils servent à développer des notes de type fleuri, fruité, vert, dans les produits dans lesquels ils sont incorporés.

## Revendications

1. Formiate de 1,3-diméthyl-but-3-ène-1-yle.
2. Propionate de 1,3-diméthyl-but-3-ène-1-yle.
3. Butyrate de 1,3-diméthyl-but-3-ène-1-yle et isobutyrate de 1,3-diméthyl-but-3-ène-1-yle.
4. Isovalérianate de 1,3-diméthyl-but-3-ène-1-yle.
5. Tiglate de 1,3-diméthyl-but-3-ène-1-yle.
6. Salicylate de 1,3-diméthyl-but-3-ène-1-yle.
7. Procéder pour améliorer, modifier ou renforcer les propriétés odorantes de parfums et produits parfumés ainsi que les propriétés gustatives d'aliments ou boissons, caractérisé en ce qu'on ajoute auxdits produits au moins un des esters des revendications 1 à 6.
8. Composition parfumante ou aromatisante caractérisée en ce qu'elle contient en tant qu'ingrédient actif au moins un des esters des revendications 1 à 6.

## Patentansprüche

1. 1,3-Dimethyl-but-3-en-1-yl-formiat.
2. 1,3-Dimethyl-but-3-en-1-yl-propionat.
3. 1,3-Dimethyl-but-3-en-1-yl-butyrat und 1,3-Dimethyl-but-3-en-1-yl-isobutyrat.
4. 1,3-Dimethyl-but-3-en-1-yl-isovalerianat.
5. 1,3-Dimethyl-but-3-en-1-yl-tiglat.
6. 1,3-Dimethyl-but-3-en-1-yl-salicylat.
7. Verfahren zum Verbessern, Modifizieren oder Verstärken der Geruchseigenschaften von Parfümen und parfümierten Produkten, sowie der Geruchseigenschaften von Nahrungsmitteln und Getränken, dadurch gekennzeichnet, daß mindestens einer der Ester gemäß Ansprüchen 1 bis 6 zu den genannten Produkten gegeben wird.
8. Eine Parfüm- oder Aromakomposition, dadurch gekennzeichnet, daß sie als aktiven Bestandteil mindestens einen der Ester gemäß Ansprüchen 1 bis 6 enthält.

## Claims

1. 1,3-Dimethyl-but-3-en-1-yl formate.
2. 1,3-Dimethyl-but-3-en-1-yl propionate.
3. 1,3-Dimethyl-but-3-en-1-yl butyrate and 1,3-dimethyl-but-3-en-1-yl isobutyrate.
4. 1,3-Dimethyl-but-3-en-1-yl isovalerianate.
5. 1,3-Dimethyl-but-3-en-1-yl tiglate.
6. 1,3-Dimethyl-but-3-en-1-yl salicylate.
7. Process to improve, modify or enhance the odorous properties of perfumes and perfumed products as well as the gustative properties of foodstuffs and beverages, characterized in that at least one of the esters of claims 1 to 6 is added to the said products.
8. Perfuming or flavouring composition characterized in that it contains as active ingredient at least one of the esters of claims 1 to 6.